# EUROPEAN PATENT APPLICATION

(11) **EP 1 190 714 A2**
(43) Date of publication of application: **27.03.2002**
(21) Application number: 01121558.9
(22) Date of filing: 10.09.2001
(51) Int. Cl.: A61K 31/4406, A61P 7/02, A61P 9/00, A61P 9/10, A61P 35/00, A61P 37/06

(54) **Method for the treatment of thromboembolic disorders in patients with aspirin resistance**

(30) Priority: 11.09.2000 GB 0022215
(71) Applicant: Schrör, Karsten, Prof. Dr., 40225 Düsseldorf (DE); Hohlfeld, Thomas, Prof. Dr., 40225 Düsseldorf (DE); Weber, Artur-Aron, Dr., 40225 Düsseldorf (DE)
(72) Inventor: Schrör, Karsten, Prof. Dr., 40225 Düsseldorf (DE); Hohlfeld, Thomas, Prof. Dr., 40225 Düsseldorf (DE); Weber, Artur-Aron, Dr., 40225 Düsseldorf (DE)
(74) Representative: Laudien, Dieter, Dr.

(57) **Abstract**

The invention relates to a method of treating and/or preventing thromboembolic disorders in a patient, who suffers from an aspirin® resistance, which method comprises administering to said patient a therapeutically effective amount of 5E-6-(3-(2-cyano-3-*tert*-butyl-guanidino)-phenyl)-6-(3-pyridyl)hex-5-enoic acid.

## Description

### Field of the invention

The invention relates to a method of treating or preventing thromboembolic disorders in a patient, who suffers from an aspirin® resistance, which method comprises administering to said patient a therapeutically effective amount of 5E-6-(3-(2-cyano-3-*tert*-butyl-guanidino)-phenyl)-6-(3-pyridyl)hex-5-enoic acid hereinbelow coded "terbogrel".

### Background of the invention

The term aspirin® resistance as used hereinbefore and hereinbelow relates to the failure of acetylsalicylic acid to prevent arterial thrombembolism (myocardial infarction, stroke, or the like) despite of continuous application of standard-dose rates (about 100 mg/day). Aspirin® resistance is a clinical problem which occurs in approximately 20-30% of treated patients. Recently it has been found that aspirin® resistance might involve enhanced expression of the inducible cyclooxygenase isoform COX-2 which we have originally demonstrated to exist in human blood platelets (Weber A.-A., Zimmermann K.C., Meyer-Kirchrath J,. Schrör K. "Cyclooxygenase-2 in human platelets as a possible factor in aspirin resistance"; Lancet 353 : 900 (1999)). In fact, patients undergoing coronary artery bypass surgery exhibited an about 15-fold increase of COX-2 protein, which was associated with a marked increase in thromboxane formation. Moreover, this increase in thromboxane formation was not prevented by conventional aspirin treatment (100 mg/day), suggesting that this aspirin® resistance might be due to the enhanced expression of the less-aspirin sensitive COX-2 isoform (Zimmermann N., Kienzle P., Weber A.-A., Gams E., Hohlfeld T., Schrör K. "Platelet aspirin resistance is associated with an increased content of cyclooxygenase-2"; Circulation 100 : I-327 (1999)).

"Terbogrel" is a pyridine derivative of formula 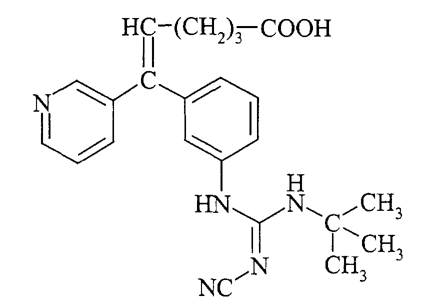 and is disclosed by US patent US 5,482,948.

It is an equipotent inhibitor of thromboxane synthase and antagonist of thromboxane receptors, being effective at low nanomolar concentrations ) (Muck S., Weber A.-A., Schrör K. (1998) "Effects of terbogrel on platelet function and prostaglandin endoperoxide transfer"; Eur. J. Pharmacol. 344 : 45 (1998)).

In addition, to blocking thromboxane-mediated actions, "terbogrel" as an inhibitor of thromboxane synthase was shown to redirect the arachidonic acid metabolism via the COX-pathway from thromboxane A₂ towards prostacyclin (PGI₂) (Muck et al, 1998, *loc. cit.*). This might result in the generation of a highly cardioprotective compound in myocardial ischemia, as demonstrated by stimulation of its endogenous biosynthesis (Hohlfeld T., Strobach H., Schrör K. "Stimulation of endogenous prostacyclin protects the reperfused pig myocardium from ischemic injury"; J. Pharmacol. Exp. Ther. 264 : 397(1993)).

However, there is no hint that this compound can be applied in order to treat or prevent thromboembolic disorders in a patient, who suffers from an aspirin® resistance.

The problem underlying the present invention was to provide a method to treat or prevent thromboembolic disorders in a patient, who suffers from an aspirin® resistance.

Surprisingly, it has been found that there is a significant inhibition of both, arachidonic acid induced platelet aggregation and expression of the adhesion molecule P-selectin (CD 62) by "terbogrel" in aspirin® resistant patients.

### Brief Description of the Invention

The invention relates to a method of treating or preventing thromboembolic disorders in a patient, who suffers from an aspirin® resistance, which method comprises administering to said patient a therapeutically effective amount of "terbogrel".

It is a further object of the invention to provide a pharmaceutical composition comprising therapeutically effective amounts of "terbogrel" and acetylsalicylic acid, and a pharmaceutically acceptable carrier and to a kit of parts, wherein a first containment (a), which comprises a pharmaceutical composition consisting essentially of therapeutically effective amounts "terbogrel" and a pharmaceutically acceptable carrier; and a second containment, which comprises a pharmaceutical composition consisting essentially of therapeutically effective amounts of acetylsalicylic acid and a pharmaceutically acceptable carrier.

Another aspect of the invention resides in the use of "terbogrel" for the preparation of a medicament for the treatment or prevention of thromboembolic disorders in a patient, who suffers from an aspirin® resistance.

### Detailed Description of the Invention

Preferred embodiments of the present invention are:
- methods for treating and/or preventing diseases in a patient in need thereof according to any of the preceding claims in which thromboxane-mediated constriction of vessels is involved, in particular reperfusion damage/ischaemia, myocardial infarction, thrombolysis, tumors, stroke or balloon dilatation, which comprises administering to said patient a therapeutically effective amount "terbogrel";
- methods for reducing severity of transplant rejection or extracorporal circulation, in particular in course of coronary bypass surgery, in a patient according to any of the preceding claims, which method comprises administering to said patient a therapeutically effective amount of "terbogrel";
- methods of treating or preventing thromboembolic disorders according to any of the preceding claims 1 in a patient, in which an increase in thromboxane formation cannot be prevented by conventional dose rates of aspirin®, which method comprises administering to said patient therapeutically effective amounts of "terbogrel" and acetylsalicylic acid.

Consequently, another aspect of the present invention are pharmaceutical compositions comprising a "terbogrel", acetylsalicylic acid and optionally one or more suitable and pharmaceutically acceptable excipients, as exemplified in: Remington: the science and practice of pharmacy. 19th ed. Easton : Mack Publ., 1995. The pharmaceutical compositions may be formulated as solids or solutions. Solid formulations may be for preparation of a solution before injection. Preferably, the pharmaceutical compositions of the invention are solutions for injection. They may be administered systemically, e.g. by intravenous injection, preferably in form of conventional intravenous preparations such as ampoules or infusions, which dosage may be administered within 5 minutes and 24 hours, or topically, e.g. by direct injection into the locus of the thromboembolic disorder. The dosage will be adjusted according to factors like body weight and health status of the patient, nature of the underlying disease, therapeutic window of the compound to be applied, solubility, and the like. It is within the knowledge of the expert to adjust dosage appropriately.

The new combination preferably contains 1 to 500 mg , preferably 2 to 75 mg of acetylsalicylic acid, and 0.01 to 300 mg, preferably 1 to 200 mg, of a compound of "terbogrel" or a physiologically acceptable addition salt thereof, incorporated together with one or more inert conventional carriers and/or diluents, e.g. corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof, can be used to produce conventional galenic preparations such as plain or coated tablets, capsules, powders, suspensions or suppositories. These are administered to adults 1 to 4 times a day, preferably 2 to 3 times a day, in order to achieve the desired effect. addition salt thereof, incorporated into conventional parenteral preparations,.
Obviously, the active substances of the above-mentioned combinations may also be administered individually, if desired. Preferably they are administered individually, in particular patients are pretreated with acetylsalicylic acid and "terbogrel" is administered thereafter.

In order that this invention be more fully understood, the following figures are set forth. These figures are for the purpose of illustrating embodiments of this invention, and are not to be construed as limiting the scope of the invention in any way.

The figures which follow are illustrative and, as recognized by one skilled in the art, particular conditions could be modified as needed for individual compositions. Materials used in tests below are either commercially available or easily prepared from commercially available materials by those skilled in the art.

### Brief Description of the Figures

- *Figure 1a:*: Shows the inhibition of platelet aggregation
- *Figure 1b:*: Shows the inhibition of platelet CD62P expression
- *Figure 2a:*: Shows the reduction of infarct size
- *Figure 2b:*: Shows the size of the ischemic area

### Detailed Description of the Figures

One skilled in the art will appreciate that although specific conditions are outlined in the following detailed explanations of the figures, modifications can be made which are meant to be encompassed by the scope of the invention. The following detailed explanations of the figures, therefore, are intended to further illustrate the invention and are not limiting.

In order to verify the effectiveness, safety and tolerability of the method of treating and/or preventing thromboembolic disorders of the present invention, randomized, placebo-controlled, double-blind parallel-group studies have been conducted in a representative sample of patients. The study was carried out in accordance with the Declaration of Helsinki and the Principles of Good Clinical Practice.

The tests have been carried out according to known method as described for example in the following documents, which are entirely incorporated herein by reference:
Schmitz G., Rothe G., Ruf A., Barlage S., Tschöpe .D, Clemetson K.J., Goodall A.H., Michelson A.D., Nurden A.T., Shankey T.V.; (1998) European Working Group on Clinical Cell Analysis: "Consensus protocol for the flow cytometric characterization of platelet function"; Thromb. Haemost. 1998 79: 885-96;
Tschoepe D., Schwippert B. "Platelet flow cytometry - adhesive proteins" in: von Bruchhausen F., Walter U.: "Platelets and their factors"; Springer, Berlin, pp. 619-643 (1997);
Weber A.-A., Strobach H., Schrör K. "Direct inhibition of platelet function by organic nitrates via nitric oxide formation"; Eur. J. Pharmacol. 247: 29-37 (1993); or
Hohlfeld T., Scharnowski F., Braun M., Schrör K. "Antiplatelet effects of ticlopidine are reduced in experimental hypercholesterolemia"; Thromb. Haemost. 71: 112-118 (1994)

The results are set forth below:

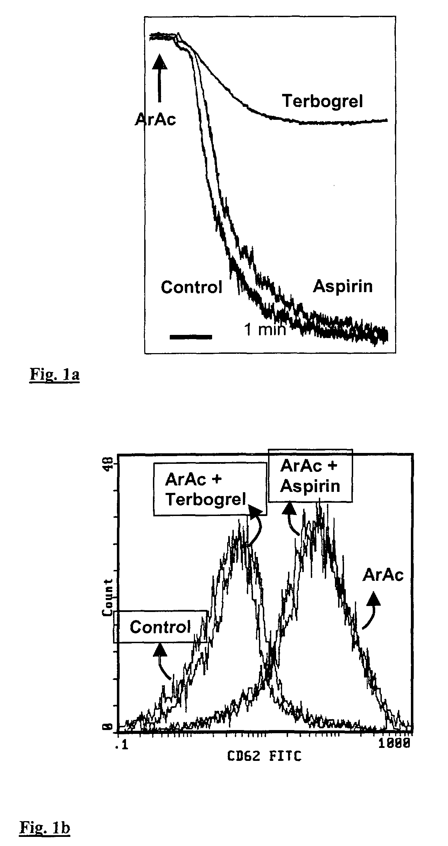

The figures show the inhibition of arachidonic acid (1 mM, ArAC) induced platelet aggregation (Fig. 1a) and platelet CD62P expression determined by flow cytometry (Fig. 1b) in platelet rich plasma obtained from an aspirin-resistant patient (day 5 after coronary bypass surgery). While acetylsalicylic acid (Aspirin) (30 µM) was ineffective to prevent aggregation and CD62P externalization, terbogrel (1 µM) almost completely inhibited both parameters of platelet activation.

Surprisingly, "terbogrel" caused a marked reduction in infarct size which was still present after acetylsalicylic acid treatment although acetylsalicylic acid itself largely prevented the beneficial effects of endogenous PGI₂ stimulation (Figure 2a). These data suggest that terbogrel in addition to its useful actions via inhibition of thromboxane synthesis and action might also redirect the metabolism of prostaglandin endoperoxides towards tissue-protective compounds (PGI₂, PGI₂) which might add to its overall biological activity.

Reduction of infarct size by "terbogrel" in pigs subjected to experimental coronary occlusion (1 h) and reperfusion (3 h), receiving an intracoronary infusion of either vehicle (control, 8 pigs) or "terbogrel" (Terbogrel, 6 pigs) (0.3 nmol/kg x min) is shown in Fig. 2a. The effect was comparable in pigs pretreated with acetylsalicylic acid (Terbogrel + ASS, 6 pigs) (1.5 mg/kg, i.v.).

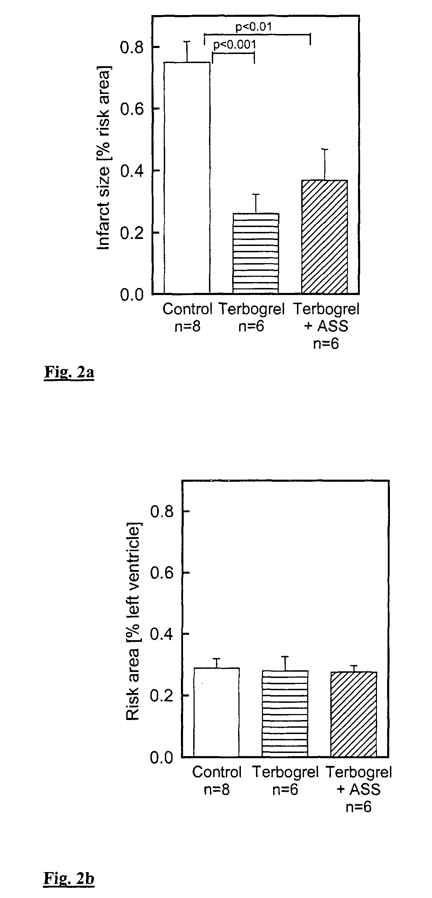

Fig. 2b shows that the size of the ischemic area was approximately 30% of the left ventricle in all experiments (Control, Terbogrel and Terbogrel + ASS).

**Conclusion -** Prevention of arterial thrombosis in patients at elevated risk and/or aspirin® resistance can be achieved with very low dose rates of "terbogrel", especially after extracorporeal circulation or patients with additional risk factors (hypertension, diabetes, previous atherothrombotic event).

## Claims

1. A method of treating and/or preventing thromboembolic disorders in a patient, who suffers from an aspirin® resistance, which method comprises administering to said patient a therapeutically effective amount of 5E-6-(3-(2-cyano-3-*tert*-butyl-guanidino)-phenyl)-6-(3-pyridyl)hex-5-enoic acid.

2. A method of treating and/or preventing thromboembolic disorders according to claim 1 in a patient, in which an increase in thromboxane formation cannot be prevented by conventional dose rates of aspirin®, which method comprises administering to said patient a therapeutically effective amount of 5E-6-(3-(2-cyano-3-*tert*-butyl-guanidino)-phenyl)-6-(3-pyridyl)hex-5-enoic acid.

3. A method according to claim 2, wherein the conventional dose rate of aspirin® is about 100 mg per day.

4. A method for treating and/or preventing diseases in a patient in need thereof according to any of the preceding claims in which thromboxane-mediated constriction of vessels is involved, which method comprises administering to said patient a therapeutically effective amount of 5E-6-(3-(2-cyano-3*-tert*-butyl-guanidino)-phenyl)-6-(3-pyridyl)hex-5-enoic acid.

5. A method according to any of the preceding claims for the treatment and/or prevention of reperfusion damage/ischaemia, myocardial infarction, thrombolysis, tumors, stroke or balloon dilatation, which method comprises administering to said patient a therapeutically effective amount of 5E-6-(3-(2-cyano-3-*tert*-butyl-guanidino)-phenyl)-6-(3-pyridyl)hex-5-enoic acid.

6. A method for reducing severity of transplant rejection in a patient according to any of the preceding claims, which method comprises administering to said patient a therapeutically effective amount of 5E-6-(3-(2-cyano-3-*tert*-butyl-guanidino)-phenyl)-6-(3-pyridyl)hex-5-enoic acid.

7. A method of treating and/or preventing thromboembolic disorders according to any of the preceding claims 1 in a patient, in which an increase in thromboxane formation cannot be prevented by conventional dose rates of aspirin®, which method comprises administering to said patient therapeutically effective amounts 5E-6-(3-(2-cyano-3-*tert*-butyl-guanidino)-phenyl)-6-(3-pyridyl)hex-5-enoic acid and acetylsalicylic acid.

8. A pharmaceutical composition comprising therapeutically effective amounts of 5E-6-(3-(2-cyano-3-*tert*-butyl-guanidino)-phenyl)-6-(3-pyridyl)hex-5-enoic acid and acetylsalicylic acid, and a pharmaceutically acceptable carrier.

9. A kit of parts for treating or preventing thromboembolic disorders in a patient, who suffers from an aspirin® resistance, which kit comprises
(a) a first containment containing a pharmaceutical composition comprising therapeutically effective amounts 5E-6-(3-(2-cyano-3-*tert*-butyl-guanidino)-phenyl)-6-(3-pyridyl)hex-5-enoic acid and a pharmaceutically acceptable carrier; and
(b) a second containment containing a pharmaceutical composition comprising acetylsalicylic acid and a pharmaceutically acceptable carrier.

10. Use of 5E-6-(3-(2-cyano-3-*tert*-butyl-guanidino)-phenyl)-6-(3-pyridyl)hex-5-enoic acid for the preparation of a medicament for the treatment or prevention of thromboembolic disorders in a patient, who suffers from an aspirin® resistance.
